# EUROPEAN PATENT APPLICATION

(11) **EP 3 527 223 A1**
(43) Date of publication of application: **21.08.2019**
(21) Application number: 18157083.9
(22) Date of filing: 16.02.2018
(51) Int. Cl.: A61K 39/12, C07K 14/005

(54) **MUTATED PARVOVIRUS STRUCTURAL PROTEIN**

(71) Applicant: 2A Pharma AB, 21134 Malmo (SE)
(72) Inventor: PRANGSGAARD, Jeanette, 9220 Aalborg Öst (DK)
(74) Representative: Banse & Steglich Patentanwälte PartmbB

(57) **Abstract**

The present invention relates to a mutated parvovirus structural protein, comprising at least one insertion comprising a sequence of at least six amino acids comprised within amino acids 320 to 641 of human HSP70i. Furthermore, the invention relates to multimeric structures comprising the protein, VLPs, a method of producing the mutated parvovirus structural protein and to medicaments or vaccines comprising the mutated parvovirus structural protein that may be used for treating vitiligo or other autoimmune diseases.

## Description

### Field of invention

The present invention relates to a mutated parvovirus structural protein, comprising at least one insertion comprising a sequence of at least six amino acids comprised within amino acids 320 to 641 of human HSP70i. Furthermore, the invention relates to multimeric structures comprising the protein, VLPs, a method of producing the mutated parvovirus structural protein and to medicaments or vaccines comprising the mutated parvovirus structural protein that may be used for treating vitiligo or other autoimmune diseases.

### Background

Vitiligo is the most frequently occurring depigmentary disorder affecting approximately 0.5 to 2% of the population worldwide. Vitiligo lesions are milky white patches that can increase in shape and size and may affect most parts of the body. The disease may develop at any age. However, half of the patients are affected by vitiligo before the age of 20 years. Vitiligo has been shown to exert a detrimental influence on the quality of life, mainly due to the change in appearance of the patients caused by the depigmentation. The disorder can affect the patients' emotional and psychological well-being. Vitiligo is associated with an increased risk of developing a depression, and more than a third of the patients with vitiligo experience some type of depressive symptoms without necessarily fulfilling all criteria for clinical depression (Speeckaert & van Geel, 2017).

Vitiligo can be differentiated into the far more common form of non-segmental vitiligo and segmental vitiligo. Non-segmental vitiligo is characterized by the development of depigmentation on both sides of the body, whereas segmental vitiligo is limited to one side of the body, usually not crossing the midline of the body. Non-segmental vitiligo usually has a chronic course with a continuing progression throughout life. In contrast, segmental vitiligo is characterized by a rapid disease onset and a disease stabilization after one to two years. Early recognition of the vitiligo subtype is essential, as the treatment is markedly different (Speeckaert & van Geel, 2017).

At present, topical corticosteroids are the established first-line treatment option for the management of vitiligo. The anti-inflammatory effects of these compounds might decrease disease progression, however, their effect on repigmentation is limited. Generally, most repigmentation can be observed in the face and neck, while only limited repigmentation is observed on the trunk, extremities and especially the hands. The side effects of corticosteroids include skin atrophy, telangiectasia and striae. Treatment is often continued for at least six months, with the primary aim to achieve a disease stabilization. As an alternative to a topical administration, corticosteroids may be administered orally at moderate doses. Oral corticosteroid therapy has been shown to stop disease progression in the majority of the patients. However, also for orally administered corticosteroids, repigmentation is only rarely observed. Furthermore, oral corticosteroid administration is associated with a variety of side effects, such as weight gain, acne, sleep disturbances, agitation, hypertrichosis and menstrual abnormalities which limit long-term use. Alternative to corticosteroids, a topical treatment of vitiligo may be based on topical immune modulators, such as tacrolimus or pimecrolimus, which attenuate T-cell activity. Similar to corticosteroids, the treatment shows most repigmentation in the face, while the results are moderate at other sides of the boy. Side effects include a burning sensation or flushing after alcohol intake, which is often observed and can be bothersome for some patients (Speeckaert & van Geel, 2017).

In addition to pharmacological treatments, phototherapy, especially narrow-band UVB phototherapy, has been established as a treatment for vitiligo. Phototherapy shows signs of repigmentation in the majority of the patients, however, complete repigmentation is only found in a minority of patients. Additionally, a relapse after discontinuation of phototherapy is frequently observed (Speeckaert & van Geel, 2017). In summary, the established therapies for vitiligo have a limited efficacy, are frequently associated with side effects, and usually have to be administered over a prolonged period of time, such as weeks or months.

Based on the observation that expanding vitiligo lesions are frequently infiltrated by cytotoxic T-cells directed against melanocyte differentiation antigens, such as Mart-1 or GP100, vitiligo is considered as an autoimmune disease. It has further been shown that psychological as well as chemical or mechanical stress contributes to the autoimmune aetiology of the vitiligo (Mosenson et al., 2013; Speeckaert & van Geel, 2017). Accordingly, the involvement of heat-shock proteins (HSP), especially of the inducible isoform of HSP70 (HSPi), in the cellular mechanisms underlying vitiligo has been examined. Human HSP70i is also known as HSP70A1A or HSP70A1B, which are characterized by the same amino acid sequence, but are yet encoded by separate genes with different regulatory regions. HSP70i is generally considered as a cytoplasmic protein, similar to HSC70, but is also secreted by living cells in contrast to the other members of the HSP70 protein family (Mosenson et al., 2013). HSP70 family proteins are known to be involved in the activation and maturation of dendritic cells (DCs), in the antigen presentation by the DCs and in the activation of T-cells. HSP70 contributes to the process of antigen presentation by dendritic cells by a) forming a complex with a peptide antigen, b) delivering the peptide to the antigen-presenting cell and transferring the peptide into the cell, c) intracellulary chaperoning the antigen for MHC class I presentation. On the surface of the antigen-presenting cell, the HSP70-antigen complex may either bind to signaling receptors, such as TLR2, TLR4 and CD91 or to scavenger receptors, such as LOX-1, SREC-1, FEEL-1/CLEVER-1 or CD91. Binding of the HSP-antigen complex to signaling receptors may activate the cytokine production of the antigen-presenting cells, whereas binding to scavenger receptors results in receptor-mediated endocytosis of the complex. This stimulatory effect of HSP70 is mediated by the C-terminal domain of the protein (Malyshev, 2013).

As shown by Mosenson et al., HSP70i is expressed at significantly higher levels in the lesional or perilesional skin in comparison to non-lesional skin. Furthermore, vitiligo melanocytes secreted significantly more HSP70i in response to oxidative stress, in comparison to control melanocytes in agreement with the assumed stress-related function of HSP70i in the autoimmune aetiology of vitiligo (Mosenson et al., 2014).

The role of HSP70i in vitiligo was also confirmed by different animal models. It could, for example, be shown that vaccination of mice with a eukaryotic expression plasmid encoding the melanocyte differentiation antigen TRP-2 significantly increased depigmentation when administered in combination with a plasmid encoding HSP70i, whereas vaccination with a plasmid encoding only the melanocyte differentiation antigen alone increased depigmentation to a significantly lower extent. Notably, the effect of HSP70i was not diminished by HSP70i antibodies expressed in response to the vaccination (Denman et al., 2009). A significantly increased depigmentation was also observed in mice vaccinated with a plasmid encoding TRP-2 in combination with a plasmid encoding the C-terminal region (amino acids 320 to 641) of HSP70i. In contrast thereto, depigmentation was hardly increased upon vaccination with a plasmid encoding TRP-2 in combination with a plasmid encoding the N-terminal region (amino acids 1 to 377) of HSP70i (Mosenson et al., 2013).

Within the C-terminal region of HSP70i, the peptide sequence QPGVLIQVYEGER seems to be required for the activation of dendritic cells. The respective sequence is homologous to the DnaK peptide QPSVQIQVYQGEREIAAHNK (DnaK amino acids 407 to 426) which is known to drive dendritic cell activation during inflammation in response to infection. HSP70i variants comprising the amino acid exchange Q435A (HSP70i_{Q435A}), V438K and I440A (HSP70i_{V438K,I440A}) or V442A and Y443V (HSP70i_{V442A,Y443V}) in the respective peptide sequence exhibit significantly decreased depigmentation effects in the above-described mouse vaccination model (Mosenson et al, 2013). Furthermore, in the early and rapidly depigmenting mouse strain h3TA2, which expresses T-cells bearing a human tyrosinase-reactive TCR transgene and HLA-A2.1, vaccination with a plasmid harboring HSP70i_{Q435A} resulted in a restoration of pigmentation in contrast to mice vaccinated with an empty vector. In mice vaccinated with wild-type HSP70i, a persistent skewing of the DC phenotype towards the inflammatory subset was observed, while conversely in mice vaccinated with HSP70i_{Q435A}, a skewing towards the tolerogenic phenotype was observed. Analysis of the humoral immune response to HSP70i revealed that only antibodies that bind downstream of the QPGVLIQVYEGER peptide were generated (Mosenson et al., 2013).

In addition to vitiligo, the role of HSP70i in antigen presentation and DC activation is considered to be involved in the aetiology of many other autoimmune and/or inflammatory diseases, for example skin diseases, such as psoriasis and lupus erythematosus (Wang et al., 2011; Jacquemen et al., 2017), autoimmune diabetes (Millar et al., 2003) and graft-versus-host disease or multiple sclerosis (Mansilla et al., 2012).

Several therapeutic approaches for the treatment of autoimmune diseases, especially of vitiligo, based on HSP70i are suggested in the art.

WO 2009/036349 A1 discloses fusion proteins comprising a trimerizing domain and at least one polypeptide, such as an antibody or fragment thereof, that binds to the HSP70i polypeptide QPGVLIQVYEGE. Furthermore, the use of said fusion protein for treating vitiligo is suggested. However, specific antibodies or therapeutic effects obtained by administering antibodies or the disclosed fusion protein are not provided. Based on the dendritic cell activating properties of the C-terminals of HSP70i, the document furthermore suggests the use of a fusion protein comprising a trimerizing domain and the HSP70i polypeptide QPGVLIQVYEGE for use as a vaccine for the treatment of cancer, especially for the treatment of melanoma.

WO 2013/033395 A1 discloses a DNA vaccine for treating and altering diseases, especially vitiligo, comprising a plasmid encoding full-length HSP70i. Especially, the use of a plasmid encoding HSP70i_{Q435A} is suggested. However, for these types of DNA vaccines, activation of oncogenes as a result of genomic incorporation of the immunizing DNA is a major safety concern. Furthermore, anti-DNA antibodies might be elicited upon DNA vaccination.

WO 2009/008719 A2 discloses the use of peptides derived from HSP70 members that have been eluted from MHC class II molecules for the treatment of inflammatory or autoimmune diseases. The inflammatory diseases include Crohn's disease, granulomatous colitis, lymphocyte colitis, collagenous colitis, ulcerative colitis and coeliac disease. The autoimmune diseases include arthritis, atherosclerosis, multiple sclerosis and myasthenia gravis, rheumatoid arthritis, psoriatic arthritis and juvenile arthritis. However, vitiligo is not disclosed. The disclosed peptides include peptides from the N-terminal region of HSP70i and peptides derived from amino acids 419 to 436 and 435 to 460 of the C-terminal region. However, no experimental data regarding these peptides are disclosed.

In summary, the treatment options for vitiligo or other autoimmune diseases with an HSP70-related aetiology disclosed in the prior art suffer from limited therapeutic efficacy, significant side effects or safety concerns.

### Problem underlying the invention

In view of the prior art, it was the general problem underlying the present invention to provide active agents, compositions, methods and uses to overcome the above-mentioned disadvantages of the prior art. Especially, agents, compositions and methods suitable for treating or preventing autoimmune diseases with an HSP70i-related aetiology, especially vitiligo, should be provided. Furthermore, the agents and compositions should be conveniently administrable, safe and easy to manufacture.

### Disclosure of the invention

Surprisingly, it was found that the problem underlying the invention is solved by the mutated parvovirus protein, compositions, uses and methods according to the claims. Further embodiments of the invention are outlined throughout the description.

In a first aspect, the invention relates to a mutated parvovirus structural protein, comprising at least one insertion comprising a sequence of at least six amino acids comprised within amino acids 320 to 641 of human HSP70i.

Surprisingly, the inventive parvovirus structural protein induces high titer antibodies against human HSP70i.

A "mutated" parvovirus structural protein within the present invention is a which comprises at least the insertion comprising a sequence of at least six amino acids comprised within amino acids 320 to 641 of human HSP70i in comparison the respective wild-type parvovirus structural protein. The mutated parvovirus structural protein may comprise additional mutations, such as substitutions, insertions, and/or deletions as described in the following.

According to the present invention, "HSP70i" refers to the human inducible heat shock protein 70 also known as HSP72, HSP70A1A or HSP70A1B, which are characterized by the same amino acid sequence, but are yet encoded by separate genes with different regulators regions. The amino acid sequence of HSP70i is equivalent to the sequence of Gene Bank accession no. AQY76873.1. The respective amino acid sequence is designated SEQ ID No. 1 in the context of the present invention.

### SEQ ID NO 1:

MAKAAAIGID LGTTYSCVGV FQHGKVEIIA NDQGNRTTPS YVAFTDTERL
1 50
IGDAAKNQVA LNPQNTVFDA KRLIGRKFGD PVVQSDMKHW PFQVINDGDK
51 100
PKVQVSYKGD TKAFYPEEIS SMVLTKMKEI AEAYLGYPVT NAVITVPAYF
101 150
NDSQRQATKD AGVIAGLNVL RIINEPTAAA IAYGLDRTGK GERNVLIFDL
151 200
GGGTFDVSIL TIDDGIFEVK ATAGDTHLGG EDFDNRLVNH FVEEFKRKHK
201 250
KDISQNKRAV RRLRTACERA KRTLSSSTQA SLEIDSLFEG IDFYTSITRA
251 300
RFEELCSDLF RSTLEPVEKA LRDAKLDKAQ IHDLVLVGGS TRIPKVQKLL
301 350
QDFFNGRDLN KSINPDEAVA YGAAVQAAIL MGDKSENVQD LLLLDVAPLS
351 400
LGLETAGGVM TALIKRNSTI PTKQTQIFTT YSDNQPGVLI QVYEGERAMT
401 450
KDNNLLGRFE LSGIPPAPRG VPQIEVTFDI DANGILNVTA TDKSTGKANK
451 500
ITITNDKGRL SKEEIERMVQ EAEKYKAEDE VQRERVSAKN ALESYAFNMK
501 550
SAVEDEGLKG KISEADKKKV LDKCQEVISW LDANTLAEKD EFEHKRKELE
551 600
QVCNPIISGL YQGAGGPGPG GFGAQGPKGG SGSGPTIEEV D
601

The C-terminus of HSP70i is underlined in SEQ ID NO. 1.

According to the present invention, a sequence of at least six amino acids "comprised within amino acid 320 to 641 of HSP70i" is a sequence of at least six consecutive amino acids that constitute a part of the amino acid sequence within amino acids 320 to 641 of HSP70i.

In a preferred embodiment, the sequence of at least six amino acids comprises in the insertion are comprised within amino acid 378 to 641 of HSP70i.

The at least one insertion comprising a sequence of at least six amino acids comprised within amino acids 320 to 641 of human HSP70i may also comprise at least 7, at least 8, at least 9, at least 10, at least 11, at least 12, at least 13, at least 14, at least 15, at least 16, at least 17, at least 18, at least 19, at least 10 or at least 21 amino acids. In a further preferred embodiment, the insertion may comprise a sequence of 6 to 40, 10 to 30, or 12 to 25 amino acids, preferably 13 to 18 and most preferably 15 to 17 amino acids. Further to these amino acids, the insert might further comprise N- and C-terminal linker sequences as described below.

Since it is an object of the present invention that the mutated parvovirus structural protein induces the generation of antibodies against HSP70i, the amino acid sequence of the insertion comprises a B-cell epitope. A "B-cell epitope" is the part of a macromolecule that is recognized by the immune system, specifically by antibodies or B-cells. A B-cell epitope can be both a linear amino acid sequence and a structural epitope defined by the surface of the macromolecule which can be built by a secondary structure of amino acids or in combination with other organic substances.

In a preferred embodiment, the amino acid sequence of the insertion may be a sequence of amino acids which correspond to a sequence which is at least partially displayed on the surface of native HSP70i. Preferably, the amino acids are at least partially displayed on the surface of HSP70i in a conformation wherein a substrate polypeptide is bound to HPS70i.

The structure of the C-terminal domain of HSP70i has been solved (Zhang et al., 2014) and can be analyzed with respect to the position of the amino acid sequence of interest.

In a preferred embodiment, the amino acid sequence comprised in the insertion comprises an amino acid sequence which is involved in the activation of antigen-presenting cells, especially dendritic cells, by human HSP70i. The involvement of an amino acid sequence within human HSP70i in the activation of antigen-presenting cells may be tested by analyzing the effect of antibodies that bind to the respective sequence, or by analyzing the effect of mutation introduced into the respective sequence of HSP70i, on the dendritic cell activation by HSP70i.

An example for a suitable assay is described in Example 1 of this application. The activation of dendritic cells by HSP70i can, for example, be analyzed by the activation of immature dendritic cells in *in vitro* cell culture assays as disclosed by Mosenson et al. (2013). In case an antibody which binds to the respective sequence, or a mutation introduced into the respective sequence, inhibits the activation of dendritic cells by HSP70i, the respective sequence is considered to be a "sequence which is involved in the activation of antigen-presenting cells" within the meaning of the present invention.

As disclosed by Mosenson et al. (2013), amino acids 435 to 445 of HSP70i having the amino acid sequence APGVLIQVYEG (SEQ ID No. 2) are involved in the activation of antigen-presenting cells, for example dendritic cells. Therefore, it is a preferred embodiment of the invention that the insertion in the mutated parvovirus structural protein comprises the sequence of amino acids 435 to 445 of HSP70i with the amino acid sequence QPGVLIQVYEG (SEQ ID No. 2). Most preferably, the insertion in the mutated parvovirus structural protein comprises the sequence of amino acids 430 to 450 of HSP70i, having the sequence TYSDNQPGVLIQVYEGERAMT (SEQ ID No. 3). In a specific embodiment of the invention, the insertion in the mutated parvovirus structural protein does not comprise an amino acid sequence of at least six amino acids comprised within the amino acids 291 to 304 and/or 445 to 460 of human HSP70i.

In a further embodiment, the amino acid sequence comprised in insertion comprises at least one mutation in comparison to the corresponding sequence within HSP70i. A "corresponding sequence within HSP70i" is the sequence from which the sequence of the insertion can be derived by introducing mutations. The insertion comprising at least one mutation may have at least 50%, at least 55%, at least 60%, at least 65%, at least 70%, at least 75%, at least 80%, at least 85%, at least 90% or at least 95% amino acid sequence identity to an amino acid sequence within amino acids amino acids 320 to 641 of HSP70i, preferably to amino acids 430 to 450, or amino acids 435 to 445 of HSP70i.

In the context of the present invention, a mutation within an amino acid sequence or in a nucleotide sequence may be at least one substitution, insertion or deletion. In a substitution, at least one amino acid or nucleotide is exchanged against another amino acid or a nucleotide in the mutated sequence in comparison to the respective wild type or comparator sequence. In an insertion, at least one amino acid or nucleotide is inserted into the mutated sequence in comparison to the respective wild type or comparator sequence. In a deletion, at least one amino acid or nucleotide is omitted in the mutated sequence in comparison to the respective wild type or comparator sequence.

The substitution may be a conservative amino acid substitutions in the primary sequence. One skilled in the art will understand that the term "conservative substitution" is intended to embrace the act of replacing one or more amino acids of a protein or peptide with an alternative amino acid with similar properties and which does not substantially alter the physical-chemical properties and/or structure of function of the native protein. Analogues of this type are also encompassed within the scope of this invention. In one embodiment, substitute amino acids may be selected from other members of the class to which the amino acid belongs. For example, non-polar (hydrophobic) amino acids include alanine, leucine, isoleucine, valine, glycine, proline, phenylalanine and tryptophan. Polar neutral amino acids include serine, threonine, cysteine, tyrosine, asparagine and glutamine. The positive charged (basic) amino acids include arginine, lysine and histidine. The negative charged (acidic) amino acids include aspartic acid and glutamic acid. Examples of preferred conservative substitutions include Lys for Arg and vice versa to maintain a positive charge; Glu for Asp and vice versa to maintain a negative charge; Ser for Thr so that a free OH is maintained; and Gln for Asn to maintain a free NH₂.

In a preferred embodiment, the amino acid sequence comprised in the insertion may comprise amino acids 435 to 445, more preferably amino acids 430 to 450, of HSP70i with at least one mutation in comparison to the corresponding sequence within HSP70i. Preferably, the mutation is at least one amino acid substitution. The at least one mutation may also be two substitutions or three substitutions or more substitutions. Preferably, the at least one mutation in the insertion corresponds to the substitution Q435A (a substitution of Q in position 435 against A), the combined substitution of V438K and I440A, or the combined substitution V442A and Y443V, in the corresponding HSP70i sequence. Most preferably, the mutation is a substitution which corresponds to the substitution of Q435A in HSP70i. Thus, the insertion may preferably comprise the amino acid sequence APGVLIQVYEG (SEQ ID No. 4), more preferably the amino acid sequence TYSDNAPGVLIQVYEGERAMT (SEQ ID No. 5). As disclosed above, the aforementioned mutations eliminate the property of HSP70i to activate dendritic cells (Mosenson et al., 2013).

It is a preferred embodiment of the invention that the epitope constituted by the mutated sequence comprised in the insertion of the mutated parvovirus structural, upon administration to a subject, induces the generation of antibodies that bind to the corresponding epitope within HSP70i. The corresponding epitope within HSP70i is at least partially constituted by the amino acid sequence within HSP70i which corresponds to the sequence comprised in the insertion. An epitope which induces antibodies that bind to an epitope constituted by a different amino acid sequence represents a "mimotope". Thus, according to the invention, the mutated amino acid sequence comprised in the insertion represents a mimotope of the corresponding sequence within HSP70i.

The use of an insertion with a mutated sequence in comparison to the corresponding sequence within HSP70i may be an especially advantageous embodiment of the present invention. It is an object of the present invention that the mutated parvovirus structural protein elicits an antibody response against HSP70i in the subject administered with the parvovirus protein. However, antigenic sequences within HSP70i exhibit "self"-antigens to the human immune system. In a process termed central tolerance, B-cells that are reactive to self-antigens undergo a negative selection and are thus deleted to a large extent during the cell maturation. In the embodiment wherein the amino acid sequence of the insertion comprised in the parvovirus structural protein comprises a mutation in comparison to the respective sequence within HSP70i, the epitope of the insertion deviates from the self-antigen. Thus, the probability that B-cells, which are reactive to the insertion and cross-reactive to the self-antigen within human HSP70i, have not been depleted by the mechanism of central tolerance may be increased. Therefore, the use of a mutated amino acid sequence may increase the probability to induce antibodies against HSP70i upon administration of the parvovirus structural protein to a subject to be treated.

Furthermore, the use of a mutated HSP70i sequence as disclosed above, prevents an activation of dendritic cells by the parvovirus protein according to the invention. Thus, the administration of a parvovirus protein comprising an insertion with a mutated HSP70i sequence as disclose above may not promote the autoimmune disease to be treated through the activation of dendritic cells.

The parvovirus structural protein according to the invention may be derived from an adeno-associated virus (AAV), Goose parvovirus, Duck parvovirus, Snake parvovirus, feline panleukopenia virus, canine parvovirus, B19 or minute virus of mice (MVM) and may be mutated as described herein. Due to the high conservation of genome organization amongst the parvoviruses, the invention can easily be transferred to other parvovirus members. Preferably structural protein according to the invention may be derived from a parvovirus that shares the general capsid assembly from viral proteins VP1, VP2 and VP3. Structural proteins derived from these viruses are generally advantageous since they enable a virus-like particle (VLP) production only from VP3 as described below. Presently known viruses of this subgroup include adeno-associated virus (AAV), Goose parvovirus, Duck parvovirus, and Snake parvovirus. Preferably AAV is selected from the group consisting of bovine AAV (b-AAV), canine AAV (CAAV), mouse AAV1, caprine AAV, rat AAV, avian AAV (AAAV), AAV1, AAV2, AAV3b, AAV4, AAV5, AAV6, AAV7, AAV8, AAV9, AAV10, AAV11, AAV12, and AAV13, especially AAV2.

In a preferred embodiment, the mutated parvovirus protein is derived from AAV2. The human immune system in general is well adapted to AAV2 capsid proteins as the largest fraction of the human population is infected with this virus that is not associated with any disease. Further, AAV2 as a gene therapy vector has been tested in large number of human patients and appeared not to be associated to immunological complications or other safety concerns. Accordingly, compared to other backbones aiming at put B-cell epitopes into a multimeric structure, AAV2 has the enormous advantage that the backbone itself, for most of the vaccinated humans, will not generate an unprecedented immune reaction that may cause autoimmune diseases in vaccinated humans.

The mutated parvovirus structural protein according to the present invention may be capable of forming a multimeric structure, wherein the insertion is located on the surface of said multimeric structure. The multimeric structure may for example be a capsomer, a virus-like particle (VLP) or a virus. Epitopes presented by ordered, multivalent, highly repetitive, and often rigid structures of viruses or VLPs can lead to a strong stimulation of B-cells and the induction of robust and long-lasting antibody responses due to extensively crosslink B-cell receptors. The strong signalling may even override B-cell tolerance mechanisms, allowing the induction of potent antibody responses against self-antigens (Frietze et al., 216; WO 2008/145401 A2). Thus, the use of proteins that multimerize into highly repetitive, rigid structures, like parvovirus structural proteins according to the present invention, is especially advantageous for generating antibodies against self-antigens, such as HSP70i.

In a preferred embodiment of the present invention, the parvovirus mutated structural protein is a mutated VP3 protein. It was previously shown (WO 2010/099960 A2) that multimeric structures useful as vaccines can be generated based upon multimeric structures consisting essentially of VP3. The use of multimeric structures comprising only a single structural protein is generally considered advantageous, since clinical development of vaccines based on multimeric structures is simplified for products based on a single active compound/protein and being as pure as possible. With respect to e.g. VLPs this is a problem in general, as viruses are often composed of more than one protein and are capable of packaging specifically viral DNA or unspecifically DNA from the host cell. Accordingly, it is desirable to obtain "pure" VLPs that contain as few different proteins as possible and preferably no nucleic acid. Further, vaccines containing VP1, VP2 and VP3 are generally produced in the presence of the parvoviral Rep protein. Rep does not only represent a further protein that is attached to VLPs but also is held responsible for packaging of virus genomes and unspecific DNA into preformed capsids (King et al., 2001). Packaging of DNA is to be avoided as VLPs potentially can enter cells of a patient and thereby transfect such contaminating DNA, which may cause all sorts of unwanted effects.

Virus-like particles comprising a mutated parvovirus structural protein derived from VP3, which is not N-terminally extended by at least parts of the VP3 sequence, as the only structural protein may be obtained by expressing a mutated parvovirus structural protein derived from VP3 in a cell under control of a Rep-independent promoter. Additionally, a polypeptide designated "assembly activating protein" (AAP) is expressed according to methods as disclosed in Sonntag et al., 2010 or WO 2010/099960 A2, which allows for high yields, e.g. approximately about 10⁵, preferably about 10⁶, and more preferably about 10⁷ virus particles to be formed per cell. The mutated parvovirus structural protein derived from VP3 of a certain virus type may preferably be co-expressed with the corresponding AAP protein from said virus type (Sonntag et al., 2010 or WO 2010/099960 A2). Alternatively an AAP from a closely related virus type may be used. The sequence encoding AAP may be provided either in *cis* or in *trans* to assemble capsids consisting essentially of VP3. Virus particle titers can be quantified from lysates of transfected cells (see above) in their undiluted form or in a dilution using a commercially available titration ELISA kit which is based on the binding of the monoclonal antibody A20 to the viral capsid in an assembled state to measure the virus concentration. Since the antibody A20 does not bind to the capsid of e.g. a different virus serotype, particle titers can be visualized by electron microscopy and quantified by counting. To analyze protein expression and estimate its amount cell lysates of identical portions of transfected cells can be processed for SDS-PAGE. Upon gel electrophoresis and transfer to a nitrocellulose membrane, proteins can be probed using binders specific to the target protein (e.g. monoclonal antibodies B1, A69, anti-GFP). The amount of protein translation can be estimated from the amount of binders that specifically bind to the protein. These complexes can be visualized and quantified by e.g. immunohistochemical staining, immunofluorescent staining or radioactive labeling.

In alternative to obtaining the virus-like particles from cell lysates, as disclosed by Sonntag et al., 2010 or WO 2010/099960 A2, the virus-like particles may preferably be obtained from culture supernatant. Obtaining virus-like particles from the culture supernatant advantageously supersedes the cell lysis step in the manufacturing and facilitates the purification of the particles.

It is preferred according to this invention that the insertion(s) is (are) inserted into one or more positions selected from the group consisting of I-261, I-266, 1-381, I-447, I-448, I-453, I-459, I-471, I-534, I-570, I-573, I-584, I-587, I-588, I-591, I-657, I-664, I-713 and I-716, preferably I-261, I-453, I-534, I-570, I-573 and I-587, more preferably I-453, I-534 and I-587, especially I-453 and I-587. The used nomenclature I-### refers to the insertion site with ### naming the amino acid number relative to the VP1 protein of AAV-2, however meaning that the insertion may be located directly N- or C-terminal, preferably directly C-terminal of one amino acid in the sequence of 5 Amino acids N- or C-terminal of the given AA, preferably 3, more preferably 2, especially 1 AA(s) N- or C-terminal of the given AA. For parvoviruses other than AAV-2 the corresponding insertion sites can be identified by performing an amino acid alignment or by comparison of the capsid structures, if available. Such alignment has been performed for the parvoviruses AAV-1, AAV-2, AAV-3b, AAV-4, AAV-5, AAV-6, AAV-7, AAV-8, AAV-10, AAV-11, b-AAV, GPV, B19, MVM, FPV and CPV (Figure 3 of WO 2008/145401 A2).

The amino acid position after which the insertion was introduced and which named the site is underlined. It is also possible likewise to introduce an insertion into the five directly adjacent Amino acids located next to the underlined AA, because these are likewise located within a loop in the AAV2 capsid. For example the insertion site I-587 corresponds to an insertion before and/or after one of the following Amino acids indicated by emphasis: FQSSS TDPAT in AAV1, LQRGN₅₈₇ RQAAT in AAV2, LQSSN TAPTT in AAV3b, LQSSS TDPAT in AAV6, LQAATAAQT in AAV7, LQQQN TAPQI in AAV8, LQQAN TGPIV in AAV10, NQNAT TAPIT in AAV11, and NQSST TAPAT in AAV5.

Further, the insertion site I-453 corresponds to an insertion directly N- or C-terminal of the following ten Amino acids each, preferably directly C-terminal of the amino acid indicated by emphasis QNQSG SAQNK in AAV1, NTPSG₄₅₃ TTTQS in AAV2, GTTSG TTNQS in AAV3b, QNQSG SAQNK in AAV6, SNPGG TAGNR in AAV7, GQTTG TANTQ in AAV8, QSTGG TQGTQ in AAV10, LSGET NQGNA in AAV11 and FVSTN NTGGV in AAV5.

In a preferred embodiment the parvovirus mutated structural protein of the invention comprises two or more insertions, each comprising at least one amino acid sequence of at least six amino acids comprised within amino acids 320 to 641 of HSP70i and each inserted at a different insertion site of the parvovirus mutated structural protein, preferably wherein one insertion is at I-587 and one at I-453. The two or more insertions of sequences of at least six amino acids comprised within amino acids 320 to 641 of HSP70i may be the same sequences or different sequences. Preferably, the sequences are the same sequences, most preferably comprising at least the sequence APGVLIQVYEG.

In addition to the insertion of an amino acid sequence of at least six amino acids comprised within amino acids 320 to 641 of HSP70i, or mutants thereof, having a length as described above, the insertion may additionally preferably comprise on its N- and/or C terminus a linker sequence which preferably has a length of 2 to 10, more preferably 3 to 6 amino acids, Preferably the linker comprises or consists of small neutral or polar amino acids (A, G, S, C), which support the inserted epitope to be well accessible to the immune system. C has the advantage that two C on both sides of the linker may be able to form a hydrogen bond. Therefore, it is envisaged that both the N-terminal and C-terminal linker contain at least one C. Generally, it is preferred that the linker sequence(s) is (are) composed of A, G and S.

In a further preferred embodiment of the invention none of the 5 amino acids directly adjacent to the insertion is R and none of the amino acids of the linker, if present, is R. R in close proximity to the insertion reduces yield of the mutated structural protein/the multimeric structures composed of the mutated structural protein during expression and purification, and therefore is preferably avoided. Accordingly, the Rs at position 585 and 588 for AAV2 have been substituted for example by A. Accordingly, the parvovirus mutated structural protein comprises one or more additional mutations selected from an insertion, a deletion, a N- or C-terminal fusion of a heterologous amino acid sequence and a substitution, particularly a single-amino-acid exchange, or a combination of these, preferably a mutation of R585 of AAV2 and/or R588 of AAV2, especially a single-amino-acid exchange R585A of AAV2 and/or R588A of AAV2.

Additionally, the insertion of epitopes at position I-453 of AAV2 as described in WO 2008/145401 A2 leads to the generation of an R within the linker downstream of the insertion (see example 6.4.3, page 103, lines 12 and 14) due to the generation of useful a endonuclease restriction site. Parvovirus mutated structural proteins where this R was substituted for a small neutral or polar amino acid (in the examples for S in a R453S mutant) lead to considerably higher yield of VP3 only AAV virus-like particles (AAVLPs) during expression and subsequent purification. Therefore, it is preferred, that the linkers, if present, do not contain an R, especially that the linker directly downstream of the inserted epitope at I-453 does not contain an R.

In a further aspect, the invention relates to a multimeric structure comprising parvovirus mutated structural proteins as described above, particularly comprising at least 5, preferably at least 10, more preferably at least 30, most preferably at least 60 structural protein. Such multimeric structure may be a capsomer, a virus-like particle (VLP) or a virus. Capsomers are multimeric subunits of a viral capsid, typically consisting of 5-6 capsid proteins (pentamers and hexamers). VLPs are empty viruses, meaning that they do not comprise genetic material such as a viral genome or relevant part thereof. Alternative to ordered structures like capsomers, a virus-like particles (VLPs) or a viruses, the multimeric structures may be aggregates with amorphous structures with no symmetric order. Preferably the insertion comprising a sequence of at least six amino acids comprised within amino acids 320 to 641 of HSP70i, or mutants thereof, is located on the surface of the multimeric structure.

Another embodiment of the present invention relates to a nucleic acid coding for a parvovirus mutated structural protein of the invention such as DNA, RNA, mRNA etc. A further embodiment of the present invention is a vector, e.g. a virus that comprises a nucleic acid encoding the parvovirus mutated structural protein of the invention. Such virus may be infectious or inactive, for example it may have been inactivated through standard techniques such as attenuation or irradiation.

In a further embodiment, the present invention is a cell comprising a nucleic acid coding for the parvovirus mutated structural protein as described above. Such cell can be a bacterium, preferably *E. coli,* a yeast cell, preferably *s. cerevisiae, hansenula polymorpha* or *pichia pastoris, k. lactis,* an insect cell, preferably SF-9, SF+ or High5, or a mammalian cell, preferably HeLa, 293, VERO, PERC6, BHK or CHO.

The parvovirus mutated structural proteins of the invention can be prepared by a method comprising the steps of:
a) producing the structural protein by cultivating the cell according to the invention under suitable conditions thereby expressing the nucleic acid of the invention, and optionally co-expressing a nucleic acid encoding an assembly activating protein (AAP), and
b) optionally isolating the expressed parvovirus mutated structural protein produced in step a).

In a preferred embodiment, essentially only VP3 is expressed, leading to multimeric structures comprising essentially only VP3. Expression and purification according to this method may for example be performed in accordance with Example 1 of this application. Expression of parvovirus mutated structural proteins comprising an insertion and purification of the obtained AAVLPs is furthermore disclosed in WO 2012/031760 A1, Example 1, for mammalian cells or by WO 2010/099960 A2, Example 1, for insect cells.

Another subject of the invention relates to a composition comprising at least one parvovirus mutated structural protein according to the invention and/or a nucleic acid according to the invention, and/or preferably at least one multimeric structure according to the invention.

In a further aspect, the invention relates to a parvovirus mutated structural protein according to the invention and/or a nucleic acid according to the invention, preferably a multimeric structure according to the invention, for use as a medicament. Furthermore, the invention relates to a composition comprising at least one parvovirus mutated structural protein according to the invention and/or a nucleic acid according to the invention, preferably at least one multimeric structure according to the invention, for use as a medicament.

The medicament may preferably be used as a vaccine comprising at least one parvovirus mutated structural protein of the invention and/or a nucleic acid of the invention, preferably at least one multimeric structure of the invention.

The medicament and/or vaccine may preferably be for use in a method for treating or preventing an autoimmune and/or inflammatory disease or in a method of immunosuppression. The autoimmune and/or inflammatory disease may be selected from vitiligo, aleopecia, arthritis, especially rheumatoid arthritis, psoriasis, lupus erythematosus, multiple sclerosis, Parkinson's disease, autoimmune diabetes (type 1 diabetes), graft versus host,host versus graft reaction Neuromyelitis optica (NMO), Acute optic neuritis (AON), coophorytis, and tumors expressing HSP70. The method of immunosuppression may preferably be a method wherein an immunoreaction in a subject against transplanted tissue, especially against a transplanted organ, is suppressed. Within the context of the present invention, "treating or preventing" a disease or condition, relates to the application of a compound or composition as described herein, to (a) preventing the disease or condition or symptom thereof from occurring in a subject which may be predisposed to and/or may acquire the disease or condition or symptom thereof, but has not yet been diagnosed as having it; (b) inhibiting the disease or condition symptoms, i.e. arresting its development; or (c) relieving or eliminating the disease or condition symptoms, i.e. causing regression of the disease or condition or symptoms thereof. For vitiligo, the symptoms are the depigmentation of skin as described above.

In a preferred embodiment, the composition, medicament or vaccine encompasses pharmaceutically acceptable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the (poly)peptides herein disclosed. In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol, citric acid or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The composition, medicament or vaccine may further comprise an immunostimulatory substance such as an adjuvant. The adjuvant can be selected based on the method of administration and may include mineral or plant oil-based adjuvants, Montanide incomplete Seppic adjuvant such as ISA, oil in water emulsion adjuvants such as the Ribi adjuvant system, syntax adjuvant formulation containing muramyl dipeptide, or aluminum salt adjuvants. Preferably, the adjuvant is an oil-based adjuvant, preferably ISA206 (SEPPIC, Paris, France), most preferably ISA51 or ISA720 (SEPPIC, Paris, France). In another preferred embodiment the parvovirus mutated structural protein is co-formulated with at least one suitable adjuvant such as CpG, Imidazoquinolines, MPL, MDP, MALP, flagellin, LPS, LTA, or cholera toxin or derivative thereof, saponins, QS21, ISCOMs, CFA, SAF, MF59, adamantanes, aluminum hydroxide, aluminum phosphate or a cytokine.

In a more preferred embodiment, the immunostimulatory substance is selected from the group comprising polycationic polymers, especially polycationic peptides such as polyarginine, immunostimulatory deoxynucleotides (ODNs), peptides containing at least two LysLeuLys motifs, especially KLKLLLLLKLK, neuroactive compounds, especially human growth hormone, alumn, adjuvants or combinations thereof. Preferably, the combination is either a polycationic polymer and immunostimulatory deoxynucleotides or of a peptide containing at least two LysLeuLys motifs and immunostimulatory deoxynucleotides. In a still more preferred embodiment the polycationic polymer is a polycationic peptide. In an even more preferred embodiment of the invention the immunostimulatory substance is at least one immunostimulatory nucleic acid. Immunostimulatory nucleic acids are e.g. neutral or artificial CpG containing nucleic acids, short stretches of nucleic acids derived from non-vertebrates or in form of short oligonucleotides (ODNs) containing non-methylated cytosine-guanine dinucleotides (CpG) in a defined base context (e.g. as described in WO 96/02555). Alternatively, also nucleic acids based on inosine and cytidine as e.g. described in WO 01/93903, or deoxynucleic acids containing deoxy-inosine and/or deoxyuridine residues (described in WO 01/93905 and WO 02/095027) may preferably be used as immunostimulatory nucleic acids in the present invention. Preferably, mixtures of different immunostimulatory nucleic acids are used in the present invention. Additionally, the aforementioned polycationic compounds may be combined with any of the immunostimulatory nucleic acids as aforementioned. Preferably, such combinations are according to the ones described in WO 01/93905, WO 02/32451, WO 01/54720, WO 01/93903, WO 02/13857 and WO 02/095027 and the AU application A 1924/2001.

In a preferred embodiment, the composition, medicament or vaccine may not comprise a further immunostimulatory substance such as an adjuvant as described above. Advantageously, the AAV backbone itself has a strong immune stimulatory property.

The composition, medicament or vaccine according to the invention may be administered to a subject in need thereof, preferably a mammal, most preferably a human, in any conventional manner, including different routes, e.g. by intravenous, intraperitoneal, intra-lymph node, subcutaneous, intradermal, intramuscular, topical, intranasal or intrabronchial administration. Preferably, the composition, medicament or vaccine is administered subcutaneous or intramuscular.

The volume of each dose for administration is preferably up to about 5 ml, still more preferably between 1 ml and 3 ml, and most preferably about 2 ml. The volume of the dose when intramuscular injection is the selected administration route is preferably up to about 5 ml, preferably up to 3 ml, preferably between 1 ml and 3 ml, more preferably between 0.5 ml and 2 ml, and most preferably about 1 ml. The amount of vaccine in each dose should be enough to confer effective immunity against HSP70i protein and decrease the risk of developing clinical signs associated with the autoimmune disease the patient is suffering from or has a chance of developing, or prevents or reverts organ transplant rejection to a subject receiving a vaccination therewith.

Preferably, the unit dose of protein or nucleic acid should be up to about 5 µg protein/kg body weight, more preferably between about 0.2 to 3 µg, still more preferably between about 0.3 to 1.5 µg, more preferably between about 0.4 to 0.8 µg, and still more preferably about 0.6 µg. Alternative preferred unit doses could be up to about 6 µg protein or nucleic acid/kg body weight, more preferably between about 0.05 to 5 µg, still more preferably between about 0.1 to 4 µg.

The dose is preferably administered 1 to 4 times, especially 1 to 3 times, e.g. with an interval of 1 to 3 months. Preferred amounts of protein per dose are from approximately 1 µg to approximately 1 mg, more preferably from approximately 5 µg to approximately 500 µg, still more preferably from approximately 10 µg to approximately 250 µg and most preferably from approximately 25 µg to approximately 100 µg.

In still a further embodiment the invention relates to a method for vaccination and/or for treating or preventing the diseases specified herein by administering to a patient, preferably a mammal, most preferably a human, an effective amount of a parvovirus mutated structural protein, nucleic acid, composition, medicament or vaccine according the invention. Accordingly, the parvovirus mutated structural protein, composition or vaccine according to the invention can be used in a method of preventing or treating an autoimmune and/or inflammatory disease. The autoimmune and/or inflammatory disease may be selected from vitiligo, aleopecia, arthritis, especially rheumatoid arthritis, psoriasis, lupus erythematosus, multiple sclerosis, Parkinson's disease, autoimmune diabetes (type 1 diabetes), graft versus host, host versus graft reaction Neuromyelitis optica (NMO), Acute optic neuritis (AON), oophorytis, and tumors expressing HSP70.

An "effective amount" of a parvovirus mutated structural protein, nucleic acid, composition, medicament or vaccine may be calculated as that amount capable of exhibiting an in vivo effect, e.g. preventing or ameliorating a sign or symptoms. Such amounts may be determined by one of skill in the art.

Above mentioned problems are solved by the invention as claimed herein.

Surprisingly, the inventive parvovirus structural protein induces high titer antibodies against human HSP70i, especially against a sequence within HSP70i which is involved in the activation of dendritic cells. Since the activation of dendritic cells by HSP70i is involved in the aetiology of autoimmune diseases, especially in the aetiology of vitiligo, the parvovirus structural protein is suitable for the treatment and/or prevention of autoimmune diseases, especially vitiligo. The use of VLPs comprising the parvovirus structural protein is especially advantageous, since these VLPs induce high antibody titers. In contrast to the administration of corticosteroids that is associated with significant side effects, the administration of VLPs is usually not associated with side effects. Furthermore, in contrast to the present treatments of vitiligo, which have to be frequently administered over a prolonged period of time, vaccination with VLPs comprising the respective parvovirus structural protein usually only requires very few administrations.

The present invention shall be explained in more detail by the following figures and examples.

### Examples

### Example 1: Generation of AAVLP-HSP70i VLPs

### 1.1 Cell Lines and Culture Conditions

Human embryonic kidney (HEK) 293T cells were cultivated in T175 flasks and maintained in Dulbecco Modified Eagle Medium (DMEM) supplemented with 10% heat-inactivated fetal-calf serum, 100 U of penicillin/mL, and 100 µg of streptomycin/mL at 37°C in 5% CO₂.

### 1.2. Cloning of AAVLP-HSP70i

AAVLPs were generated from a plasmid containing overlapping AAV2 VP2 and VP3 coding sequences cloned into the *Xhol*I and *Not*I site of the pCI plasmid (Promega, Madison, WI). The start codon of VP2 was destroyed by introducing a point-mutation using the Quick Change Site-Directed Mutagenesis kit (Agilent Technologies, La Jolla, CA) to generate the plasmid pCIVP2mutACG. The point-mutation resulted in a ACG to GAG mutation. In order to introduce peptides into the VP3, the plasmid pCIV2mutACG was modified. The plasmid pCIVP2mutACG-I587 was generated by introduction of *Not*I and *Bsp*EI sites at position 587. The plasmid pCIVP2mutACG-I453 was generated by introduction of *Not*I and *Bsp*EI sites at position 453. Afterwards, yet another point-mutation was introduced using the Quick Change Site-Directed Mutagenesis kit to destroy an additional *Not*I site within the backbone of the pCI vector generating the plasmid pCIVP2mutACG_mut*Not*I-I587 and the plasmid pCIVP2mutACG_mut*Not*I-I453.

The nucleotide sequence of wildtype residues 430 to 445 (TYSDNQPGVLIQVYEG) of HSP70i and mutated residues 430 to 445 (TYSDNAPGVLIQVYEG) of HSP70i was cloned into either the *Not*I /*Bsp*EI digested pCIVP2mut ACG_mut*Not*I-I587 or the *Not*I /*Bsp*EI digested pCIVP2mutACG_mut*Not*I-I453 to generate four different plasmids for the AAVLP-HSP70i production.

### 1.3 Production and Purification of AAVLP-HSP70i

HEK293T cells were transfected with AAVLP-HSP70i plasmid DNA (36 µg per T175 flask mixed with PEI I (1:4)) in serum free DMEM + 1% P/S. Supernatant was collected after 3-4 days and the medium was cleared by filtration, diluted three times in dilution buffer (15 mM Sodium Citrate, 6mM EDTA, 0.001%F-68, pH 5.5±0.3) and adjusted to pH 6.0. Particles were further purified through chromatography. Briefly, the cleared supernatant containing the AAVLPs were loaded onto a Capto S column (GE Healthcare) and after washing with buffer A containing (10mM Sodium Citrate, 50mM NaCl, 2mM EDTA, 0.001%F-68, pH 6.0±0.3) a gradient elution from 0-30 % was applied with buffer B (50mM TrisHCl, 1M NaCl, 2mM EDTA, 0.001%F-68, pH 8.5±0.3) and fractions were collected during this gradient. The AAVLP-HSP70i particles were analysed by SDS-PAGE and the purity was determined by Western Blotting. The titer was determined using the AAV2 Titration ELISA.

### 1.4 SDS-PAGE and Western Blotting

Fractions of purified AAVLP-HSP70i particles was analysed and identified by SDS-PAGE and coomassie blue staining. Expression and purity of the AAVLP-HSP70i VP3 proteins was verified by Western blotting using an antibody (to be decided). The blotted membrane will be incubated with 5% skim milk in 1xPBS/0.1% Tween-20 for 1 hour at RT followed by incubation of the membrane with antibody (to be decided) for 1 hour at RT. After washing, bound antibodies will be detected with 1:20,000 diluted HRP-labeled anti-X IgG analysed by Odyssey® FC imaging system (LiCor, Lincoln, USA).

### 1.6 Capsid Titer Determination by AAV2 Titration ELISA

Capsid titer in HEK293T cells as described under 1.3 may be determined using a commercially available AAV2 titration ELISA kit (Progen, #PRATV) according to the manufacture's manual. Briefly, the particles are serial diluted and incubated in a 96-well plate coated with mouse monoclonal antibody to AAV2 for 1 hour at 37°C. After washing, the captured AAVLP-HSP70i particles are incubated with an anti-AAV2 biotin-conjugated monoclonal antibody for 1 hour at 37°C. The washing is repeated and a streptavidin peroxidase conjugate is added to react with the biotin molecule followed by incubation for 1hour at 37°C. After washing, a substrate solution is added resulting in a colour reaction, which is proportional to the amount of specifically bound viral particles. A stop solution is added after 15 minutes of incubation at RT. The absorbance (OD) is measured photometrically using an ELISA reader at 450 nm. A kit control containing AAV2 particles is included and serial diluted in two-fold resulting in a typical titration curve. The curve allows quantitative determination of the AAVLP-HPS70i capsid titer.

### 1.6 In Vitro Activation of Immature DCs

*In Vitro* dendritic cell (DC) activation assay may be performed according to the following protocol. DCs will be derivated from monocytes isolated from human peripheral blood mononuclear cells (PBMCs). The PBMCs will be isolated on different occasions from whole blood of healthy volunteers by density gradient centrifugation with lymphoprep (Axis-Shield). Monocytes are isolated from PBMCs by negative selection using magnetic beads according to manufacturer's instructions (Miltenyi). To generate DCs, the cells are maintained in Teflon containers (Savillex) to prevent adherence in RPMI medium (Mediatech Inc.) supplemented with 10% fetal bovine serum, penicillin (100 U/ml), streptomycin (100 mg/ml), recombinant human GM-CSF (100 ng/ml) (Leukine, Berlex), and recombinant human IL-4 (25 ng/ml) (R&D Systems). On day 7, either LPS (Sigma) or HSP70i (1 µg/ml) (BioVision) are added. After 24 hours, expression of activation markers is assessed by flow cytometry.

The *in vitro*-generated human DCs from PBMCs are gently harvested and stained with different antibodies to detect the maturation of DCs e.g. mouse anti-human CD11c, CD80, CD83, CD86, and HLA-ABC antibodies labelled with fluorescent markers (BD Biosciences). Initial gating was performed on live non-debris singlets, with subsequent gating toward immature and matures markers using FACSCanto II equipment (BD Biosciences).

### 1.7 Measurement of Antibody Responses

Rats will be immunized with AAVLP HSP70i at day 0 and booster vaccines are given 1 and 3 months. Sera will be taken from the animals 2 weeks after each vaccination. The presence of wildtype HSP70i-specific and mutated HSP70i-specific IgG and IgM antibodies in sera of immunized rats will be determined by an ELISA. Streptavidin pre-coated immobilizer plates (Nunc, Thermo Scientific) were coated overnight at 4°C with 1µg/well of two HSP70i-specific biotinylated peptides. The plates were then blocked with 5% skim milk in 1xPBS/0.1% Tween-20 for 1 hour at RT followed by incubation with a dilution series of animal sera for 1 hour at 37°C. After washing with 1xPBS/0.1% Tween-20 the bound AAVLP-HSP70i antibodies were incubation with 1:20,000 diluted HRP-labels anti-animal antibody. The enzymatic reaction was detected by adding substrate (TBM) resulting in a colour reaction, which intensity measured in OD value was analysed using an ELISA reader at 450 nm.

### References

Denman CJ, McCracken J, Hariharan V, Klarquist J, Oyarbide-Valencia K, Guevara-Patiño JA, Le Poole IC. (2008) HSP70i accelerates depigmentation in a mouse model of autoimmune vitiligo. J Invest Dermatol. 128(8):2041-8.
Frietze KM, Peabody DS, Chackerian B. (2016) Engineering virus-like particles as vaccine platforms. Curr Opin Virol. 18:44-49
Jacquemin C, Rambert J2, Guillet S, Thiolat D, Boukhedouni N, Doutre MS, Darrigade AS, Ezzedine K, Blanco P5 Taieb A, Boniface K, Seneschal J. (2017) HSP70 potentiates interferon-alpha production by plasmacytoid dendritic cells: relevance for cutaneous lupus and vitiligo pathogenesis. Br J Dermatol. doi: 10.1111/bjd.15550
King JA, Dubielzig R, Grimm D, Kleinschmidt JA. (2001) DNA helicase-mediated packaging of adeno-associated virus type 2 genomes into preformed capsids. Embo J. 20: 3282-91.
Malyshev I (2013) Immunity, Tumors and Aging: The Role of HSP70. Dordrecht, Heidelberg, New York, London: Springer. 63-82
Mansilla MJ, Montalban X, Espejo C. (2012) Heat shock protein 70: roles in multiple sclerosis Mol Med. 18:1018-28
Millar DG, Garza KM, Odermatt B, Elford AR, Ono N, Li Z, Ohashi PS. (2003) Hsp70 promotes antigen-presenting cell function and converts T-cell tolerance to autoimmunity in vivo. Nat Med. 9(12):1469-76
Mosenson JA, Zloza A, Nieland JD, Garrett-Mayer E, Eby JM, Huelsmann EJ, Kumar P, Denman CJ, Lacek AT, Kohlhapp FJ, Alamiri A, Hughes T, Bines SD, Kaufman HL, Overbeck A, Mehrotra S, Hernandez C, Nishimura MI, Guevara-Patino JA, Le Poole IC. (2013) Mutant HSP70 reverses autoimmune depigmentation in vitiligo. Sci Transl Med. 5(174):174ra28
Mosenson JA, Flood K, Klarquist J, Eby JM, Koshoffer A, Boissy RE, Overbeck A, Tung RC, Le Poole IC. (2014) Preferential secretion of inducible HSP70 by vitiligo melanocytes under stress. Pigment Cell Melanoma Res. 27(2):209-20
Sonntag F, Schmidt K, Kleinschmidt JA. (2010) A viral assembly factor promotes AAV2 capsid formation in the nucleolus. Proc Natl Acad Sci USA. 107(22):10220-5
Speeckaert R, van Geel N. (2017) Vitiligo: An Update on Pathophysiology and Treatment Options. Am J Clin Dermatol. doi: 10.1007/s40257-017-0298-5. PMID: 28577207
Wang D, Eiz-Vesper B, Zeitvogel J, Dressel R, Werfel T, Wittmann M.(2011) Human keratinocytes release high levels of inducible heat shock protein 70 that enhances peptide uptake. Exp Dermatol. 20(8):637-41

## Claims

1. A mutated parvovirus structural protein, comprising at least one insertion comprising a sequence of at least six amino acids comprised within amino acids 320 to 641 of human HSP70i.

2. The mutated parvovirus structural protein according to claim 1, wherein the amino acid sequence comprised in the insertion comprises an amino acid sequence which is involved in the activation of antigen-presenting cells by HSP70i.

3. The mutated parvovirus structural protein according to any of the preceding claims, wherein the amino acid sequence of the insertion comprises at least one mutation in comparison the corresponding sequence in HSP70i.

4. The mutated parvovirus structural protein according to any of the preceding claims, wherein the amino acid sequence of the insertion comprises the amino acid sequence APGVLIQVYEG and/or QPGVLIQVYEG.

5. The mutated parvovirus structural protein according to any of the preceding claims, wherein the mutated parvovirus structural protein is derived from AAV, preferably AAV2.

6. The mutated parvovirus structural protein according to any of the preceding claims, wherein the mutated parvovirus structural protein is a mutated VP3 protein.

7. The mutated parvovirus structural protein according to any of the preceding claims, wherein the mutated parvovirus structural protein comprises two or more insertions according to any of the preceding claims.

8. The mutated parvovirus structural protein according to any of the preceding claims, wherein the insertions are at positions I-587 and/or I-453.

9. The mutated parvovirus structural protein according to any of the preceding claims, wherein the mutated parvovirus structural protein comprises a linker sequence, and/or
comprises one or more additional mutations selected from an insertion, a deletion, a N- or C-terminal fusion of a heterologous amino acid sequence and a substitution.

10. A multimeric structure, preferably a virus-like particle, comprising a mutated parvovirus structural protein according to any of the preceding claims.

11. A nucleic acid encoding a mutated parvovirus structural protein.

12. A composition comprising the mutated parvovirus structural protein according any of the preceding claims for use as medicament.

13. The composition according to claim 12, wherein the medicament is a vaccine.

14. The composition according to claims 12 to 13, wherein the medicament and/or vaccine is for use in a method for treating or preventing an autoimmune and/or inflammatory disease or in a method of immunosuppression.

15. The composition according to claim 14, wherein the autoimmune and/or inflammatory disease is selected from vitiligo, aleopecia, arthritis, especially rheumatoid arthritis, psoriasis, lupus erythematosus, multiple sclerosis, Parkinson's disease, autoimmune diabetes, graft versus host host versus graft reaction, and Neuromyelitis optica (NMO), Acute optic neuritis (AON), oophorytis, and tumors expressing HSP70.
